Europäisches Patentamt

European Patent Office

Office européen des brevets

(19)

(11) Veröffentlichungsnummer: **0 236 377 B1**

(12) # EUROPÄISCHE PATENTSCHRIFT

(45) Veröffentlichungstag der Patentschrift: **03.06.92**

(51) Int. Cl.5: **G02B 27/00**, A61F 9/00, B23K 26/10

(21) Anmeldenummer: **86905214.2**

(22) Anmeldetag: **11.09.86**

(86) Internationale Anmeldenummer:
**PCT/DE86/00360**

(87) Internationale Veröffentlichungsnummer:
**WO 87/01819 (26.03.87 87/07)**

(54) **VORRICHTUNG ZUR ERZEUGUNG EINES LASERSTRAHLFLECKS EINSTELLBARER GRÖSSE.**

(30) Priorität: **11.09.85 DE 3532464**
**12.03.86 DE 3608242**

(43) Veröffentlichungstag der Anmeldung:
**16.09.87 Patentblatt 87/38**

(45) Bekanntmachung des Hinweises auf die
Patenterteilung:
**03.06.92 Patentblatt 92/23**

(84) Benannte Vertragsstaaten:
**AT CH DE FR GB IT LI**

(56) Entgegenhaltungen:
**EP-A- 0 143 743       EP-A-00 151 869**
**GB-A- 2 111 716       US-A- 4 213 618**
**US-A- 4 443 075       US-A- 4 538 608**
**US-A-41 758 32**

(73) Patentinhaber: **G. RODENSTOCK INSTRUMEN-
TE GMBH
Drachenseestrasse 10-12
W-8000 München 70(DE)**

(72) Erfinder: **REIS, Werner
Dachauerstrasse 407
W-8000 München 50(DE)**
Erfinder: **BISLE, Werner
Karwendelstrasse 21
W-8027 Neuried(DE)**

(74) Vertreter: **Schiller, Walter, Dr. et al
Kanzlei Münich, Steinmann, Schiller Willibaldstrasse 36-38
W-8000 München 21(DE)**

EP 0 236 377 B1

## Beschreibung

Die Erfindung bezieht sich auf eine Vorrichtung zur Durchführung von Koagulationen auf dem Augenhintergrund gemäß dem Oberbegriff des Patentanspruchs 1. Eine solche Vorrichtung ist aus US-A-4 538 608 bekannt.

Bei Koagulationen auf den Augenhintergrund stellt sich allgemein das Problem, den Laserstrahl, der je nach Art des Lasers einen bestimmten Querschnitt hat, der zu behandelnden Fläche anzupassen.

Hierzu werden in der Regel strahlaufweitende optische Elemente in Verbindung mit Elementen, beispielsweise Varioobjektiven verwendet, die die Vergrößerung ändern. Auf diese Weise ist es beispielsweise möglich, die Strahlfleckfläche des Operationslasers auf dem Augenhintergrund zwischen bestimmten Durchmessern zu varriieren. Dabei ergibt sich jedoch das Problem, daß gleichzeitig auch der Öffnungskegel des Strahls variiert wird. Wird der Öffnungskegel zu klein, so ist die Energiedichte bereits vor dem eigentlichen Operationsort so groß, daß sie Schädigungen hervorrufen kann.

Aus der US-A-4 213 678, ist es bekannt, bei einem Scanning Laser Ophthalmoskop, bei dem ein Laserstrahl den Augenhintergrund zur Erzeugung eines Bildes abtastet, den Laserstrahl zur Erzielung einer Koagulationswirkung in dem Zeitpunkt, in dem er den zu behandelnden Bereich abtastet, auf eine therapeutisch wirksame Leistung "hochzuschalten". Diese Vorgehensweise ist aber in der Praxis kaum brauchbar, da die "Verweilzeit" auf dem Augenhintergrund so gering ist, daß ein äußerst leistungsstarker Laser verwendet werden muß, um akzeptable Koagulationswirkungen zu erzielen. Deshalb hat dieser Vorschlag keine weitere Beachtung gefunden.

Ferner ist es aus der US-A 4 538 608 oder der EP-A 0 151 869 bekannt, zur Keratotomie der Cornea weitgehend beliebige Strahlfleckflächen eines Excimer-Lasers dadurch einzustellen, daß das gewünschte Schnittmuster "ausgetastet" wird. Bei der Keratotomie der Cornea spielt jedoch der Öffnungswinkel des Laserstrahls keine Rolle.

Der Erfindung liegt die Aufgabe zugrunde, eine Vorrichtung zur Durchführung von Koagulationen auf dem Augenhintergrund zu schaffen, bei der auch bei stark unterschiedlich großen Fleckgrößen beispielsweise Verletzungen an der Hornhaut oder Iris aufgrund zu hoher Strahlungsintensität sicher vermieden und bei der darüberhinaus beliebige Fleckformen erzeugt werden können.

Eine erfindungsgemäße Lösung dieser Aufgabe ist mit ihren Weiterbildungen in den Patentansprüchen gekennzeichnet.

Verbrennungen etc. von Stellen vor der zu behandelnden Stelle werden dadurch vermieden, daß mit einem Laserstrahl mit gleichbleibend großem Öffnungskegel gearbeitet wird und die zu bestrahlende Fläche auf dem Objekt nicht mit einem stationären Laserstrahl relativ hoher Energie bestrahlt wird, sondern von dem Laserstrahl nach einem vorgegebenen Muster überstrichen wird. Je nach Einstellung dieser Überstreichbewegung können somit unterschiedlich große Fleckgrößen realisiert werden. Die Größe dieser Strahlflecken kann dabei entsprechend dem gewählten Austastmuster stufenlos eingestellt werden, wodurch eine zoom-artige Verstellbarkeit gewährleistet ist. Damit kann eine Schalt-Zoomoptik eingespart werden, und es ist eine kleinere Bauweise des Adapters möglich. Dennoch werden Schädigungen wirkungsvoll verhindert, da der Öffnungswinkel des Einstrahlkegels stets gleich bleibt, und sich so die Energiedichte vor der Koagulationsstelle nicht verändert.

Bei Koagulationen am Augenhintergrund hat sich eine Laserstrahlfleckgröße von 50 $\mu$m, mit der ein größerer Laserstrahlfleck ausgetastet wird, als besonders zweckmäßig erwiesen. Je nach Art der verwendeten Optik, der Laserstrahlquelle und auch je nach Anwendungsgebiet sind selbstverständlich auch andere Fleckgrößen möglich und sinnvoll.

Insbesondere bei Augenoperationen wird bevorzugt ein kreisförmiger Strahlfleck verwendet. Hierbei ist das Verhältnis von bestrahlter zu behandelnder Fläche besonders günstig; diese Fleckkonfiguration ist ferner für den Wärmeabfluß besonders vorteilhaft. Je nach Anwendungsfall können jedoch auch andere Fleckformen beispielsweise quadratische Formen gewählt werden.

Vor allem aber erlaubt die erfindungsgemäße Vorrichtung die Einstellung eines bestimmten Energieprofils auf dem koagulierten Bereich durch die Steuerung der auf einem koagulierten Flächenelement deponierten Energie in Abhängigkeit vom momentanen Behandlungsort, so daß ein optimaler Koagulationserfolg auch bei großen zu koagulierenden Bereichen erzielt werden kann.

Die Steuerung der deponierten Energie kann insbesondere durch die in den Ansprüchen 2 oder 5 gekennzeichneten Mitteln erfolgen.

Im Anspruch 2 ist angegeben daß die Mittel zur Steuerung der deponierten Energie Mittel zur Steuerung der Abtastgeschwindigkeit in Abhängigkeit vom momentanen Abtastort des Behandlungsgebietes aufweisen.

Hieran schließen sich die Seiten 4 bis 20 der mit der Regel 51(4)EPÜ übersandten Unterlagen.

Die Austastung des Flecks kann gemäß Anspruch 2 beispielsweise zeilenförmig erfolgen. Erfindungsgemäß ist es jedoch nach Anspruch 3 auch bevorzugt, einen kreisförmigen Strahlfleck mit einem rotierenden Laserstrahl zu bearbeiten, d.h. der Laserstrahl läuft auf einer Kreisringbahn um. Durch Vergrößerung des Bahndurchmessers wird dabei die gewünschte Fleckgröße erzielt. Die Verstellung der Fleckgröße kann dabei automatisch oder manuell, ganz nach den Erfordernissen, ausgeführt werden. Die Umlaufbewegung des Laserstrahls wird vorteilhaft automatisch durchgeführt, wobei eine übliche Umlaufzeit bei etwa 8 msec. liegt. Dieser Wert ist jedoch nicht einschränkend zu verstehen.

Um eine bestimmte Verteilung der Leistung über die Strahlfleckfläche, beispielsweise eine Gauß-Verteilung oder eine homogene bzw. gleichmäßige Ausleuchtung des Strahlflecks zu erzielen, ist es zusätzlich zu den im Anspruch 1 angegebenen Merkmalen auch möglich, die Leistung des Laserstrahls ortsabhängig zu verändern (Anspruch 4).

Beispielsweise ist es zum Erzielen einer gleichmäßigen Ausleuchtung möglich, die Leistung des Laserstrahls mit zunehmenden Kreisringdurchmesser zu erhöhen (Anspruch 5). Je nach den Erfordernissen kann somit die Energie der Laserstrahlung zum Rande des Strahlflecks hin kontinuierlich verstärkt oder abgeschwächt werden.

Eine weitere Möglichkeit besteht gemäß Anspruch 6 darin, den Laserstrahl langsamer umlaufen zu lassen, so daß der längere Umlaufweg des Laserstrahls am Rande des Strahlflecks durch eine entsprechend höhere Einwirkzeit ausgeglichen werden kann.

Die Austastbewegung kann natürlich in unterschiedlichster Weise, beispielsweise durch akustooptische Ablenkelemente realisiert werden. Bevorzugt weist die erfindungsgemäße Vorrichtung jedoch eine Einrichtung mit zumindest einem bewegbaren Optikteil auf (Anspruch 7), wobei die Einrichtung vorteilhaft als Taumeleinheit (Anspruch 8) ausgebildet ist, d.h. eine gleichzeitige Bewegung um zwei Achsen ausführen kann. Bei dem oder den Optikteilen handelt es sich bevorzugt um Planplatten oder Spiegel (Ansprüche 10 bis 13).

Bei einem Ausführungsbeispiel der erfindungsgemäßen Vorrichtung sind als Optikteile zwei übereinander angeordnete schwenkbare Planplatten (Anspruch 10) vorgesehen, deren Schwenkachsen einen Winkel miteinander bilden und die phasenverschoben durch eine Antriebseinrichtung vorzugsweise hin- und herverschwenkt werden. Zweckmäßig sind die Achsen der beiden Planplatten in einem Winkel von 90° zueinander angeordnet, und die beiden Planplatten werden mittels Winkelmotoren mit um 90° zueinander verschobenen Signalen, vorzugsweise Sinus-Signalen (Anspruch 11) angetrieben. Durch diese aufeinander abgestimmte Auslenkung mittels der beiden Planplatten kann der Laserstrahl in eine kreisförmige Rotationsbewegung versetzt werden.

Bei einem anderen vorteilhaften Ausführungsbeispiel der Erfindung weist der Optikteil mindestens einen Spiegel auf. Mit zwei Spiegeln (Anspruch 18), deren Schwenkachsen einen 90°-Winkel einschließen, kann beispielsweise eine x/y-Abtastung des gewünschten Strahlflecks erzielt werden. Ferner ist es möglich, einen Spiegel zu verwenden, der um eine Schwenkachse gedreht und um eine andere Schwenkachse gekippt wird. Durch die Drehbewegung des Spiegels wird wiederum ein Rotieren des Laserstrahls erzeugt, wobei der Durchmesser des vom Laserstrahl überstrichenen Kreisrings durch Kippen des Spiegels kontinuierlich verstellt werden kann. Mittels einer Drehzahlregelung des spiegeldrehenden Motors kann die Strahlungsleistung, die auf den Strahlfleck auftrifft, kontinuierlich eingestellt werden (Ansprüche 14, 16, 17).

Bei einem weiteren Ausführungsbeispiel der Erfindung ist als optisches Element eine rotierende, kippbare Glasplatte vorgesehen (Anspruch 13), die den Laserstrahl auslenkt und in eine Kreisbewegung mit ebenfalls einstellbarem Durchmesser versetzt. Durch kontinuierliche Vergrößerung der Kippung kann selbstverständlich auch eine spiralförmige Bewegung des Laserstrahls erzeugt werden. Alternativ ist auch eine zeilenweise Abrasterung möglich.

Um eine möglichst genaue Führung des Laserstrahls zu erzielen und eine möglichst große Bewegbarkeit des Optikteils zu erreichen, ist der Optikteil vorzugsweise kardanisch aufgehängt, so daß Kippbewegungen in jeder Richtung möglich sind. Hierdurch ist eine weitgehend spiel- und reibungsfreie Lagerung ermöglicht, bei der radiale Verdrehungen verhindert sind. Erfindungsgemäß kann die Taumeleinheit eine mechanische Einrichtung sein.

Bevorzugt ist eine Ausführung der Taumelbewegung auf elektromagnetischem Wege. Dies gestattet eine Ausbildung der Anordnung mit wenigen Verschleißteilen und ermöglicht eine schnelle Einstellung der gewünschten Position des Optikteils. Der kardanisch gelagerte Teil der Einrichtung besteht aus Dauermagneten, denen gegenüberliegend entgegengesetzt wirkende Dauermagneten angeordnet sind, und den Taumelkörper in der Ruhelage stabilisieren. Auf der anderen Seite sind bevorzugt Magnetspulen angeordnet, die phasenversetzt betätigt werden und örtlich und zeitlich versetzt jeweils auf den Taumelkörper anziehende Momente ausüben. Hierdurch kann eine exakte kreisförmige Taumelbewegung des Optikteils herbeigeführt werden (Ansprüche 19 folgende).

Zweckmäßig ist eine Verlagerung des Kardangelenks der Anordnung zum Zentrum hin, so daß die beim Taumeln bewegten Massen kleingehalten werden können, was zu einer höheren mechanischen Resonanzfrequenz der Anordnung führt. Eine weitere Verbesserung in dieser Hinsicht ist eine Verringerung der bewegten Massen insgesamt. Hierdurch kann erreicht werden, daß die Taumelfrequenz in einem sicheren Abstand unterhalb der Resonanzfrequenz der mechanischen Anordnung liegt. Aufgrund der Fleckgröße und der Wirkung des Laserflecks auf der Netzhaut ergibt sich eine maximal notwendige Taumelfrequenz von etwa 20 Hz.

Es können vorteilhaft mechanische Anschläge vorgesehen sein, um zu verhindern, daß die Magnete bei großen Ausschlägen der Taumelanordnung kleben, d.h. an dem gegenüberliegenden Dauermagneten oder Polen der Elektromagnete hängenbleiben. Eine andere Maßnahme zur Lösung dieses Problems kann darin bestehen, die fest angeordneten Magneten so anzuordnen, daß sie bei maximalen Auslenkungen mit ihren Stirnflächen zueinander ausgerichtet sind. Bei einer bevorzugten Ausführungsform kann die erfindungsgemäße Vorrichtung auf drei verschiedene Arten betrieben werden: Im Handbetrieb, bei dem Taumelbewegung Schritt für Schritt nachvollzogen werden kann; halbautomatisch, wobei die Taumelbewegung jederzeit wieder angehalten bzw. alternativ der Taumelradius nachgestellt werden kann, und vollautomatisch. In der letzten Betriebsweise können beliebige Muster, bei Verwendung eines schnellen Verschlusses (Anspruch 29) auch Punktmuster, beispielsweise eine Reihe von nach einem bestimmten Muster angeordneten Punkten, deren Größe dem Durchmesser des Laserstarhls entspricht oder auch größer ist, projiziert werden.

Bei der Verwendung von Arbeitslasern, deren Licht im nicht sichtbaren Bereich des Spektrums liegt, also entweder im UV- oder Infrarot-Bereich, ist es seit längerem üblich, eine sogenannte Ziellichtquelle zu verwenden. Bei medizinischen Operationslasern wird dabei der Strahl der Ziellichtquelle deckungsgleich mit dem Strahl des Operationslasers geführt, so daß der Arzt vor der Operation überprüfen kann, ob der Strahl des Operationslasers auf den zu behandelnden Bereich eingestellt ist.

Hierbei ergibt sich jedoch das Problem, daß der Strahl der Ziellichtquelle Sensoren, beispielsweise Bildsensoren, mit denen der Fundus erfaßt wird, überstrahlt, so daß keine Einzelheiten des Fundus aufgelöst werden können.

Erfindungsgemäß ist nun erkannt worden, daß der dieser Anmeldung zugrundeliegende allgemeine Erfindungsgedanke, eine vorgebbare Fläche mit einem auf ein kleines Gebiet fokussierten Strahl auszutasten, auch eine Lösung dieses Problems erlaubt:

Im Gegensatz zu den bekannten Lösungen, bei denen der Ziellichtstrahl und der Arbeitslichtstrahl deckungsgleich geführt sind, wird erfindungsgemäß der Ziellichtstrahl auf einer Bahn bewegt, die den Strahlfleck des Arbeitslichtstrahls umgibt. Es kann damit weiterhin überprüft werden, ob der Arbeitslichtstrahl, also beispielsweise der Strahl eines Operationslasers, auf den richtigen Fleck eingestellt ist; da der Ziellichtstrahl jedoch das zu bearbeitende bzw. zu behandelnde Gebiet umgibt, werden Strukturen dieses Gebiets nicht durch das Licht des Ziellichtstrahls, beispielsweise eines Helium-Neon-Lasers (Anspruch 26) überstrahlt.

Die Bahn, auf der der Ziellichtstrahl geführt ist, kann dabei beliebig der Fleckdimensionierung des Arbeitslichtstrahls angepaßt werden. Bei Koagulationen ist es selbstverständlich möglich, den Ziellichtstrahl auf einer ringförmigen Bahn zu führen, die den Strahlfleck des Arbeitslasers ringförmig umgibt (Anspruch 27).

Die Erfindung wird im folgenden anhand bevorzugter Ausführungsbeispiele und der Zeichnung weiter erläutert. In der Zeichnung zeigen:

Fig. 1

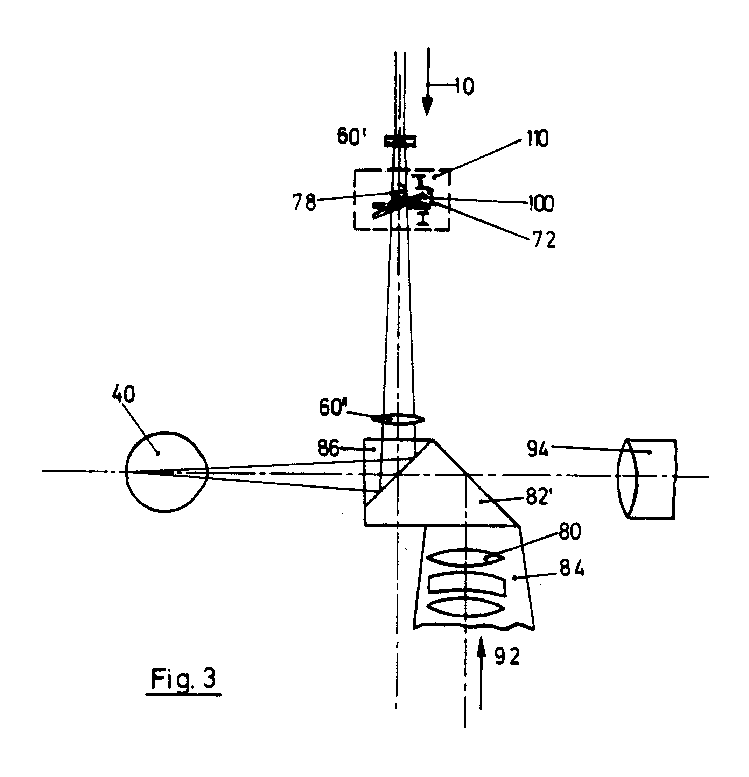

ein erstes Ausführungsbeispiel der erfindungsgemäßen Einrichtung, die zwei übereinander angeordnete schwenkbare Planplatten als Optikteile aufweist,

Fig. 2a und 2b

zwei weitere Ausführungsbeispiele der erfindungsgemäßen Vorrichtung, bei der das Optikteil ein Spiegel ist,

Fig. 3

ein weiteres Ausführungsbeispiel der erfindungsgemäßen Vorrichtung, bei der das Optikteil eine Glasplatte ist,

Fig. 4

ein Beispiel einer Halterung und Aufhängung der in Fig. 3 gezeigten Glasplatte,

Fig. 5

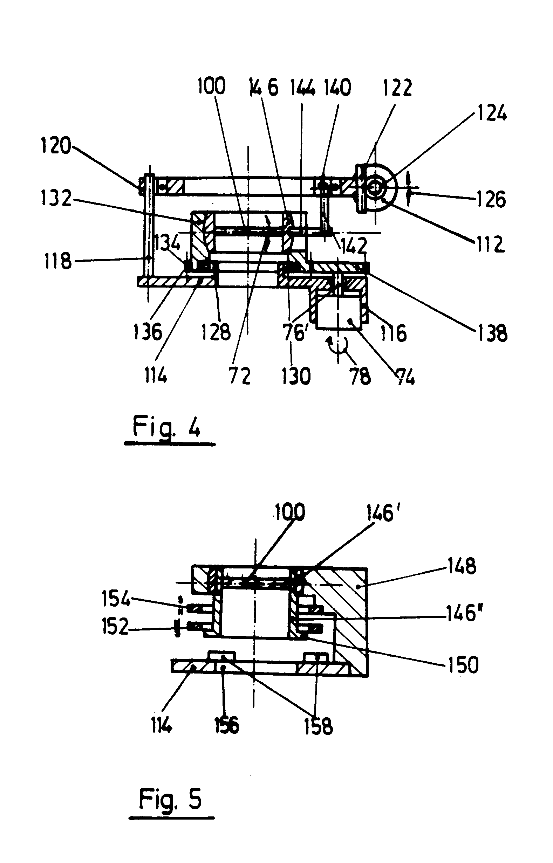

ein weiteres Beispiel für die Halterung und Aufhängung der in Fig. 3 gezeigten Glasplatte,

Fig. 6a bis 6c

detaillierte Darstellungen der in Fig. 5 gezeigten Halterungsanordnung, und

Fig. 7

eine Variante der Magnetanordnung, und

Fig. 8

ein Ausführungsbeispiel mit x/y-Abtastung.

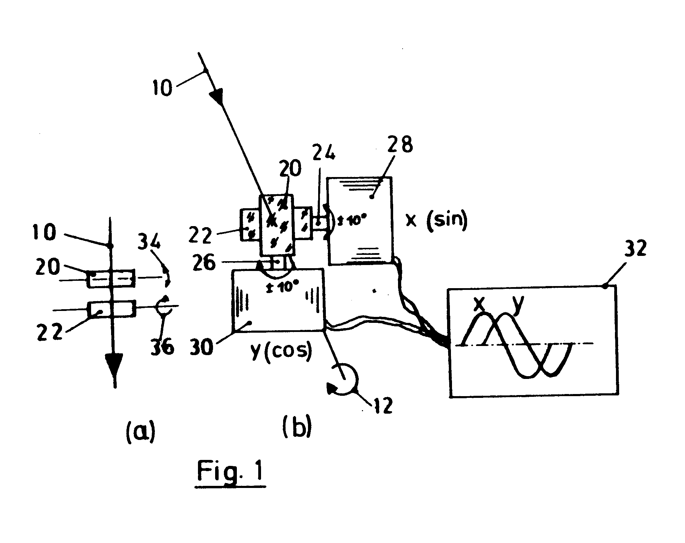

In Fig. 1 ist ein erstes Ausführungsbeispiel der erfindungsgemäßen Vorrichtung dargestellt. Im Strahlengang des Lasers, der mit dem Pfeil 10 angedeutet ist, sind zwei identische rechteckförmige Planscheiben übereinander mit Abstand voneinander angeordnet. Die beiden Planplatten 20, 22 sind dabei so übereinander angeordnet, daß sie mit einem Teil ihrer Flächen einander überlappen, wobei ihre Längsachsen einen Winkel von 90° miteinander bilden und der Laserstrahl durch den Überlappungsbereich im wesentlichen in dessen Mitte hindurchtritt. In Verlängerung ihrer Längsachsen sind die beiden Planplatten 20, 22 jeweils mit einem Wellenabschnitt 24, 26 versehen, der jeweils an einen Antriebsmotor, z.B. einen Winkelmotor 28 bzw. 30 angekoppelt ist. Im gezeigten Ausführungsbeispiel werden die beiden Planplatten um jeweils 10° aus der Horizontalen in beide Richtungen verschwenkt. Dabei wird die Achse des Wellenabschnitts 24 als X-Achse und die Achse des Wellenabschnitts 26 als Y-Achse bezeichnet. Die beiden Achsen werden mit um 90° zueinander versetzten Signalen, im gezeigten Ausführungsbeispiel mit Sinus-Signalen, gesteuert, was durch den Zusatz "sin" bzw. "cos" für Sinus und Cosinus in der Zeichnung angedeutet werden soll. Dieser Zusammenhang ist im schematischen Schaubild in Fig. 1(b) durch zwei zueinander versetzt gezeichnete Sinus-Kurven x und y veranschaulicht.

Während Fig. 1(b) als Draufsicht auf die Planplatten-Anordnung die relative Lage der Schwenkachsen 26 und 24 zueinander veranschaulicht, wird in Fig. 1(a) die unterschiedliche Verschwenkung der beiden Planplatten 20 und 22 durch die Pfeile 34 und 36 weiter erläutert. Der Pfeil 10 deutet den Strahlengang des Laserstrahls bei unbewegten Platten an. Durch die phasenverschobene Verschwenkung der beiden Planplatten wird der Laserstrahl so ausgelenkt, daß er eine kreisförmige Rotationsbewegung ausführt. Dies ist unten in Fig. 1(b) durch den kreisförmigen Pfeil 12 angedeutet.

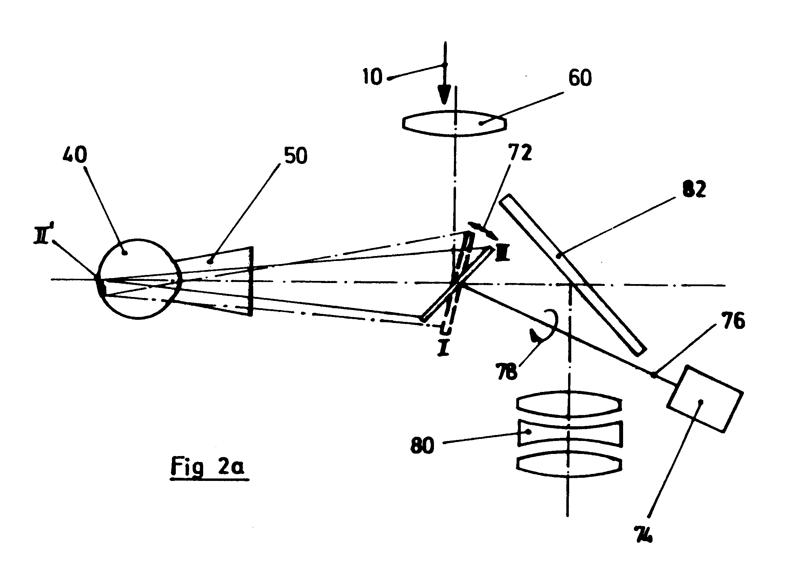

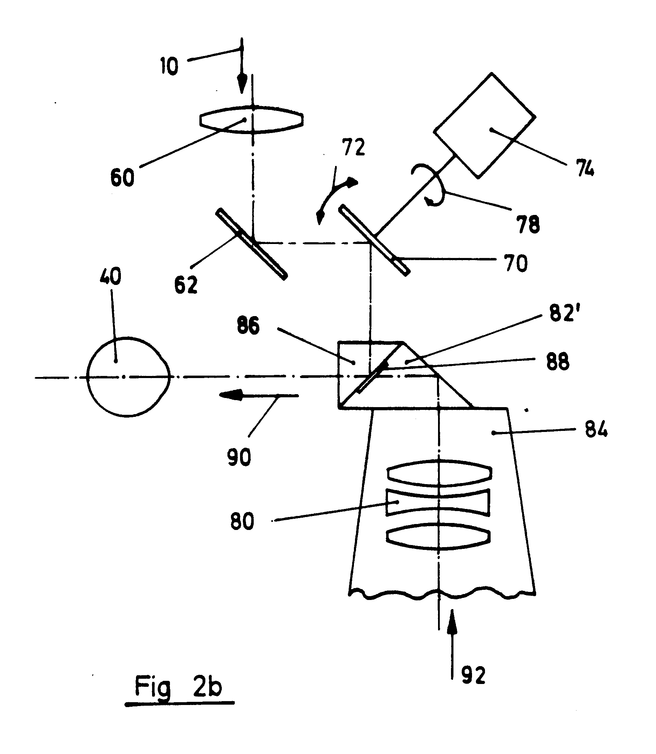

Fig. 2(a) und (b) zeigen zwei Ausführungsbeispiel der erfindungsgemäßen Vorrichtung, bei der als Optikteil ein Spiegel verwendet ist. Es wird zunächst die Fig. 2 (a) beschrieben. Eine Aufweitoptik 60 dient zur Verbreiterung des Laserstrahls. Hinter dieser Aufweitoptik ist ein rotierender, kippbarer Spiegel 70 angeordnet, der in zwei Kippositionen I und II dargestellt ist, wobei ein Pfeil 72 die Kippbewegung zwischen diesen Positionen I und II veranschaulicht. Die Aufweitoptik ist so konzipiert, daß der Laserstrahl auf dem Spiegel 70 einen Durchmesser von etwa 10 mm besitzt und auf dem Augenhintergrund des Auges 40 einen Strahlfleck von etwa 50 μm bildet. Den verschiedenen Kippositionen I und II entsprechen verschiedene Positionen des Strahlflecks in der vertikalen Richtung auf dem Augenhintergrund, was durch die Kennzeichnungen I' und II' angedeutet ist. Ein Motor 74 ist mit einer Drehachsenanordnung 76 mit dem Spiegel 70 verbunden und versetzt den Spiegel in eine sehr schnelle Drehbewegung und kippt ihn zugleich, was durch die Pfeile 72 und 78 angedeutet ist. Der Motor 74 ist drehzahl-geregelt und stufenlos verkippbar, so daß der Durchmesser der vom Laserstrahl auf dem Augenhintergrund überstrichenen Fläche kontinuierlich verstellbar ist, was eine Realisierung beliebig großer Fleckgrößen auf dem Augenhintergrund gestattet. Wird beispielsweise eine gängige Motordrehzahl von etwa 8000 Umdrehungen pro Minute gewählt, so kann eine Ringzone einmal in etwa 8 msec. überstrichen werden.

In Fig. 2 (a) ist ferner der Beleuchtungsstrahlengang schematisch dargestellt. Ein Objektiv 80 dient zur Spaltbeleuchtung. Das Beleuchtungslicht wird durch einen Umlenkspiegel 82 zum Auge hin umgelenkt und gelangt über ein Kontaktglas 50 in das Auge und beleuchtet den Augenhintergrund. Dieser Beleuchtungsstrahlengang ist herkömmlicher Art.

Fig. 2(b) zeigt ein anderes Ausführungsbeispiel mit einem Spiegel als Optikteil. Soweit gleiche Teile in der Anordnung verwendet sind, sind diese mit gleichen Bezugszeichen bezeichnet. Der Laserstrahl 10 gelangt wiederum durch eine Aufweitoptik 60 und wird dann jedoch nicht direkt auf den Spiegel 70 geworfen, sondern zunächst durch einen feststehenden Umlenkspiegel 62 um 90° auf den Spiegel 70 geworfen. Für den Antrieb des Spiegels 70 ist wiederum ein drehzahlgeregelter Motor 74 vorgesehen, so daß der Spiegel eine Drehbewegung (Pfeil 78) und eine Kippbewegung (Pfeil 72) ausführt. Von diesem Spiegel ausgehend tritt der sich drehende Laserstrahl nicht direkt ins Auge 40 ein, sondern gelangt vielmehr zunächst durch ein Prisma 86 auf einen Spiegel 88, von wo er zum Auge 40 hin reflektiert wird.

Das Beleuchtungslicht (vgl. Pfeil 92) gelangt zunächst durch einen Objektivkopf 84 und eine Objektivlinse 80 zu einem Umlenkprisma 82', wo es reflektiert wird und durch den halbdurchlässigen Spiegel 88 und das Prisma 86 zum Auge 40 hin gelangt. Nach dem Durchtritt durch das Prisma 86 treten bei diesem Ausführungsbeispiel das Laser- und Beleuchtungslicht aus im wesentlichen derselben Richtung ins menschliche Auge. Dies ist durch den Pfeil 90 angedeutet.

In Fig. 3 ist ein Ausführungsbeispiel der erfindungsgemäßen Vorrichtung veranschaulicht, bei dem das Optikteil eine Planplatte ist, die ähnlich wie der Spiegel schwenk- und kippbar ist, was durch die Pfeile 72 und 78 bzw. die Positionskennzeichnungen I und II angedeutet ist. Die Glasplatte ist in einer Anordnung 110 gehaltert und aufgehängt, was später noch näher erläutert ist. Wie bei den vorhergehenden Ausführungsbeispielen sind gleiche Teile mit gleichen Bezugszeichen gekennzeichnet. Die Aufweitoptik umfaßt in diesem Ausführungsbeispiel zwei Linsen 60' und 60''. Der Strahlenbeleuchtungsgang ist wie in Fig. 2b aufgebaut.

Zusätzlich ist ein Mikroskop 94 rechts des Prismas 82' angeordnet, von dem aus der Hintergrund des Auges 40 durch das Prisma 82' und das Prisma 86 im wesentlichen geradlinig beobachtet werden kann. Auch bei diesem Ausführungsbeispiel wird wiederum durch die Taumelbewegung der Glasplatte 100 der Laserstrahl aus seiner geradlinigen Ausrichtung ausgelenkt und in eine Kreisbewegung versetzt, deren Durchmesser durch Wahl des Kippwinkels der Glasplatte 100 einstellbar ist.

Fig. 4 zeigt ein Ausführungsbeispiel einer Halterung und Antriebsanordnung der Glasplatte 100, die in Fig. 3 dargestellt ist.

Eine Gehäuseplatte 14, die im wesentlichen kreisscheibenförmig ausgebildet ist und konzentrisch zum Strahlengang des Laserstrahls angeordnet ist, trägt an einer Außenseite in Strahlrichtung des Lasers nach unten weisende flanschartige Vorsprünge 116, die einen ersten Motor 74 halten, der zur Drehbewegung der Glasplatte 100 vorgesehen ist, was durch den Pfeil 78 angedeutet ist und noch näher erläutert wird. Auf der dem Motor 74 entgegengesetzten Seite trägt die Gehäuseplatte 114 drei um 120° zueinander versetzte Führungsstangen 118, die zur Laserstrahlquelle hin gerichtet, d.h. entgegengesetzt zum Motor 74 gerichtet sind. Im Bereich des äußeren Endes der Führungsstangen 118 ist eine parallel zur Gehäuseplatte 114 ausgerichtete, mit einer zentralen Öffnung versehene Platte 120 gehalten. An einem Randbereich trägt die Platte 120 eine Zahnstange 122. Mit dieser Zahnstange 122 befindet sich ein erstes Zahnrad 124 in Eingriff, das den Teil eines Getrietes eines zweiten Motors 112 bildet. Der Motor 112 dient dazu, die Platte 120 in der Höhe zu verstellen, was durch den Pfeil 126 angedeutet ist.

Auf der zum ersten Motor 74 entgegengesetzten Seite trägt die Gehäuseplatte 114 einen eine zentrale Öffnung begrenzenden Flanschring 128, auf dessen Außenseite ein Kugellager 130 angeordnet ist, das mit dem nach unten weisenden Flansch 114 eines zylindrischen Lagers in Eingriff steht. Das zylindrische Lager besitzt einen solchen Außendurchmesser, daß es mit Abstand bezüglich der Führungsstangen 118 innerhalb dieser angeordnet ist, und besitzt eine solche Höhe, daß es mit Abstand bezüglich der Platte 120 angeordnet ist. Der erste Motor 74 ist über eine Welle 76' mit einem zweiten Zahnrad 138 verbunden, das mit an der Außenseite des Flansches 134 des zylindrischen Lagers 132 ausgebildeten Zähnen 136 in Eingriff steht. Dreht sich somit der erste Motor 74, so hat dies zur Folge, daß sich die Lageranordnung 132 ebenfalls dreht.

Radial innerhalb der Führungsstangen 118 ist die Platte 120 mit einem Kugellager 140 versehen, in dem ein Arm 142 eines aus zwei gelenkig miteinander verbundenen Armen gebildeten Gestänges gelagert ist. Der Arm 142 ist dabei in der Ruhestellung in Fig. 4 nach unten, d.h. axial zum Laserstrahl ausgerichtet. Der zweite Arm erstreckt sich in der Ruhestellung horizontal radial nach innen in ein Lager 146, das als Pendellager ausgebildet ist. Das Pendellager 146 ist im wesentlichen zylindrisch, innen hohl ausgebildet und weist eine gewölbte Außenfläche auf, die mit einer entsprechend geformten gewölbten Innenfläche des Lagers 132 in Eingriff steht. Das Pendellager 146 ist mittels des zweiten Gestängearms 144 mit dem zylindrischen Lager 132 verbunden. Im Pendellager 146 ist die Glasplatte 100 fest angeordnet.

Die oben beschriebene Anordnung funktioniert folgendermaßen: Die Glasplatte 100 wird durch den ersten Motor 74 in Drehung versetzt, wobei der Antrieb über die Zahnräder 136, 138 erfolgt und durch die Kugellageranordnung 130 reibungsarm ist. Das Pendellager 146 der Glasplatte ist durch den als Mitnehmer dienenden zweiten Arm 144 des Gestänges mit dem rotierenden zylindrischen Lager 132 verbunden. Mittels des Gestänges 142, 144 kann die Glasplatte 10C in eine definierte Winkelstellung gebracht werden. Das Gestänge rotiert dabei mit, wobei es im zweiten Kugellager 140 gelagert ist. Dieses zweite Kugellager 140 ist mittels der Zahnstange 122 und einer Getriebeanordnung, bestehend aus dem Zahnrad 124 und dem Motor 112 in der Höhe verstellt. Hierdurch wird das Pendellager 146, das im zylindrischen Lager 132 bewegbar angeordnet ist, mehr oder weniger schräggestellt und dementsprechend wird die Glasplatte 100 in eine mehr oder weniger starke Schräglage gebracht, was durch den Pfeil 72 angedeutet ist.Die Führungsstangen 118 dienen dabei zur exakten Höhenführung der Platte 120. Mittels dieser Anordnung kann somit die Glasplatte gedreht und gekippt werden, ähnlich wie dies bei dem Spiegel in dem Fig. 2a und 2b und der Glasplatte in Fig. 3 der Fall war. Es wird somit ein rotierender Laserstrahl auf dem Augenhintergrund erzeugt, wobei durch entsprechende Kippung der Glasplatte ein unterschiedlicher radialer Abstand von der Strahlachse des nichtabgelenkten Laserstrahls erzeugt wird.

Fig. 5 zeigt eine zu der in Fig. 4 gezeigten alternative Anordnung. In einem Gehäuse 148, das eine zentrale zur Laserstrahlrichtung koaxiale Öffnung aufweist, ist ein Pendellager 146 mit kugelförmiger Außenwandung und zylindrischer Innenwandung angeordnet. Das Pendellager 146' ist dreh- und schwenkbar im Gehäuse 148 gelagert. Im Inneren des Pendellagers ist ein zylindrischer Hohlkörper fest angeordnet, der sich in axialer Richtung nach unten erstreckt und in dem in der Höhe des Pendellagers mit geringerer axialer Erstreckung die Glasplatte 100 fest gehaltert ist. Das Gehäuse 148 ist so ausgebildet, daß es das Pendellager 146' an einem vorspringenden Abschnitt trägt, so daß dieses nebst Zylinder 146'' frei beweglich ist und nirgends am Gehäuse 146 anstoßen kann. Die zylindrische Halterung 146'' ist am unteren, dem Pendellager 146' entgegengesetzten Ende mit einem nach außen weisenden Flansch 150 versehen, auf dem eine Ringmagnetanordnung 152 sitzt. Die Ringmagnetanordnung ist dabei so ausgebildet, daß der Nordpol nach oben und der Südpol nach unten weist. Axial mit Abstand oberhalb dieser Ringmagnetanordnung sitzt eine zweite Ringmagnetanordnung 154 mit umgekehrt angeordneten Polen, d.h. der Nordpol weist nach unten und der Südpol nach oben, so daß sich die beiden Ringmagnete gegenseitig abstoßen. Die zweite Ringmagnetanordnung ist dabei so angebracht, daß sie fest am Gehäuse 148 sitzt und sich nicht in Eingriff mit dem Zylinder 146'' befindet, wobei sich jedoch die beiden Ringmagnete axial übereinander befinden. Unten am Gehäuse sitzt eine zylindrische Gehäuseplatte 114, die ebenfalls eine zentrale Öffnung 156 aufweist und Spulen 158 trägt. Im gezeigten Ausführungsbeispiel sind drei Spulen in 120' Abständen auf der Gehäuseplatte 114 angeordnet, die als Spulenträger dient.

Die in Fig. 5 gezeigte Anordnung funktioniert folgendermaßen: Über die Spulen 158 wird phasenverschoben ein definierter Strom eingeprägt. Der fest mit dem Halterungszylinder 146'' verbundene Ringmagnet 152 wird dabei durch die Spulen abgestoßen. Durch 120°-Spulenanordnung wird ein kreisförmig wirkender Taumeleffekt hervorgerufen, ähnlich einem mit Drehstrom geregelten Motor. Der zweite Ringmagnet 154, der mit dem Gehäuse 148 verbunden ist, bewirkt durch seine definierte Abstoßung bezüglich des ersten Ringmagneten 152 eine Gegenkraft zu den Spulen 158. Auf diese Weise kann die Winkelstellung, d.h. die Kipposition der Glasplatte 100 genau eingestellt werden. Je nach Stärke des eingestellten Stroms bei den Spulen kann die Winkelstellung der Glasplatte über das Pendellager 146' eingestellt werden. Wiederum kann auf diese Weise ein rotierender Laserstrahl mit einstellbarem Radius erzeugt werden.

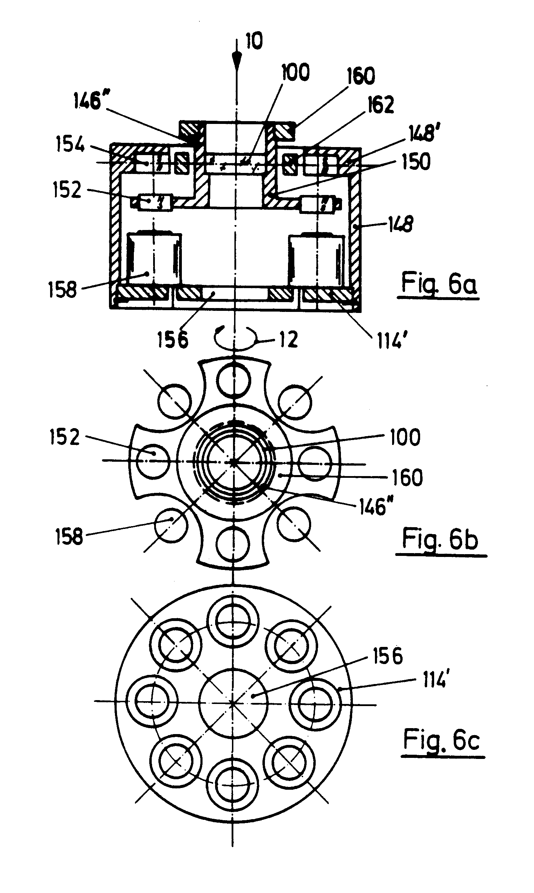

Fig. 6a bis c zeigen eine zu der Fig. 5 gezeigten alternative Anordnung zur Realisierung einer Taumelbewegung auf elektromagnetischem Wege. Die zylindrische Halterung 146'' der Planplatte 100 ist kardanisch aufgehängt, so daß Kippbewegungen in jede Richtung möglich sind. Dies ist mittels eines in Höhe der Glasplatte 100 vorgesehene Zwischenkäfigs 162 und des den Halterungszylinder 146'' mit Abstand umgreifenden Gehäuse 148 möglich. Die zylindrische Halterung 146'' weist am in Fig. 6a unteren Ende einen sich nach außen erstreckenden radialen Flansch 150 auf, der vier Dauermagnete trägt, deren Nordpole nach oben weisen. Das Gehäuse 148 weist einen sich in Höhe des Zwischenkäfigs 162 nach innen erstreckenden radialen Flansch 148' auf, der vier Dauermagnete trägt, deren Nordpole nach unten weisen und sich annähernd axial oberhalb der ersten Dauermagnete befinden. Die Dauermagnete 152, 154 halten den kardanisch gelagerten Teil der Anordnung in seiner Lage. Die vier Magnetpaare sind dabei jeweils um 90° versetzt und einander gegenüberliegend und gegensinnig gepolt angeordnet.

Axial mit Abstand von dem Flansch 150 trägt das Gehäuse 148 am unteren Ende Spulenhalterungen 114' aus z.B. Weicheisen. Auf diesen Spulenhaltern 114' sind vier um 90° versetzte Magnetspulen 158 angeordnet. Jeweils zwei einander gegenüberliegende Spulen sind dabei in Reihe geschaltet, so daß bei Stromfluß jeweils eine Spule ein anziehendes Moment auf den Taumelkörper ausübt und eine benachbarte Spule ein abstoßendes Moment. Durch die Reihenschaltung von jeweils zwei Spulen ergeben sich somit zwei Phasen, und jede Phase wird mit einem sinusförmigen Strom beaufschlagt, wobei eine Phasenverschiebung von 90° vorgesehen ist. Auf diese Weise ist bei idealen mechanisch-magnetischen Voraussetzungen eine exakt kreisförmige Taumelbewegung des Taumelkörpers erzielbar.

Die Fig. 6b und 6c stellen eine Draufsicht auf den Taumelkörper mit Ausgleichsgewicht 160 und Magneten und Glasplatte 100 von oben dar, während Fig. 6c eine Ansicht der Anordnung von unten ist.

Bei einer Schaltung zur Durchführung dieser elektromagnetisch erzeugten Taumelbewegung z.B. beim Ausführungsbeispiel der Fig. 6 muß auf zwei getrennte Kanälen ein eingeprägter sinusförmiger Strom geliefert werden, wobei Signale um 90° zueinander phasenverschoben sein müssen. Diese Phasenverschiebung darf sich selbstverständlich bei unterschiedlichen Frequenzen entsprechend einer unterschiedlich schnellen Rotation nicht verändern. Die Stromamplitude muß dabei einstellbar sein, um einen Kippwinkel beliebiger Wahl einstellen zu können.

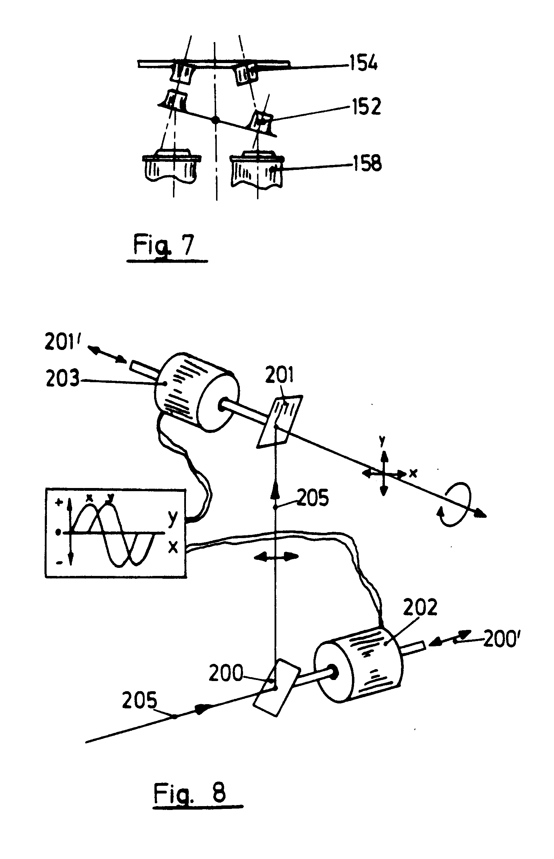

Fig 7 veranschaulicht schematisch eine andere Möglichkeit der Anordnung der feststehenden Magneten. Um ein Festkleben der Magnete zu verhindern, sind bei diesem Ausführungsbeispiel die am Gehäuse fest angeordneten Magnete 154 schräg nach außen und unten weisend angeordnet, so daß ihre Stirnflächen nach außen geneigt sind. Wenn der Taumelkörper stark auschlägt und somit die mit dem Taumelkörper fest verbundenen Magnete 152 in eine starke Schräglage geraten, dann sind sie bei der in Fig. 7 gezeigten Anordnung jedoch in einer solchen extremen Lage im Höchstfall annähernd axial zu diesen. Hierbei sind die Stirnflächen der Magnete plan zueinander ausgerichtet, und es kann zu keinem Verkleben der Magnete kommen, sondern vielmehr stoßen sich diese Magnete aufgrund der entgegengesetzten Polung ab. Ferner können die Magnete zur besseren Verteilung der Feldlinien auch schräg gestellt werden.

Fig. 8 zeigt eine weitere erfindungsgemäße Vorrichtung, die als Optikteil zwei Spiegel 200 und 201 aufweist, die von axial schwingenden Elementen 202 und 203 in Schwingungen in Richtung der Pfeile 200' bzw. 201' versetzt werden. Werden die axial schwingenden Elemente 202 und 203 mit um 90° gegeneinander verschobenen Sinus-Signalen angesteuert, so tastet der Laserstrahl 205 den gewünschten Strahlfleck ebenfalls ringförmig aus.

**Patentansprüche**

1. Vorrichtung zur Durchführung von Koagulationen auf dem Augenhintergrund, mit
einer Laserstrahlquelle,
einer Fokussiereinrichtung (60; 60'; 60'') zur Fokussierung des Laserstrahls in einem Brennfleck vorgegebener Größe auf dem Augenhintergrund, die so ausgebildet ist, daß der Öffnungswinkel des Strahlengangs in dem Bereich vor dem Fokussierungsfleck eine augenunschädliche Energiedichte der Strahlung in diesem Bereich gewährleistet,
und einer Ablenkeinrichtung (20,22;70;100;200,201) zur Erzeugung einer Abtastbewegung des Fokussierungsflecks entlang einer ausgewählten Bahn in einem ausgewählten Behandlungsgebiet auf dem Augenhintergrund,
dadurch **gekennzeichnet**, daß Mittel zur Steuerung der auf einem koagulierten Flächenelement deponierten Energie in Abhängigkeit vom momentanen Abtastort des Behandlungsgebietes vorgesehen sind.

2. Vorrichtung nach Anspruch 1,
dadurch **gekennzeichnet**, daß die Mittel zur Steuerung der deponierten Energie Mittel zur Steuerung der Abtastgeschwindigkeit in Abhängigkeit vom momentanen Abtastort des Behandlungsgebietes aufweisen.

3. Vorrichtung nach Anspruch 1,
dadurch **gekennzeichnet**, daß die Ablenkeinrichtung den Laserstrahl zeilenweise über die gewünschte Strahlfleckfläche bewegt.

4. Vorrichtung nach Anspruch 1,
dadurch **gekennzeichnet**, daß die Ablenkeinrichtung den Laserstrahl auf einer Kreisringbahn über die gewünschte Strahlfleckfläche bewegt.

5. Vorrichtung nach Anspruch 3 oder 4,
dadurch **gekennzeichnet**, daß zur Erzeugung eines vorgebbaren Leistungsdichteprofils die Mittel zur Steuerung der deponierten Energie Mittel zur ortsabhängigen Veränderung der Leistung des Laserstrahls aufweisen.

6. Vorrichtung nach Anspruch 5,
dadurch **gekennzeichnet**, daß die Leistung des Laserstrahls mit zunehmenden Kreisringdurchmesser erhöht wird.

7. Vorrichtung nach Anspruch 4 bis 6,
dadurch **gekennzeichnet**, daß die Drehzahl der Umlaufbewegung des Laserstrahls mit wachsendem Kreisringdurchmesser herabgesetzt wird.

8. Vorrichtung nach einem der Ansprüche 1 bis 7,
dadurch **gekennzeichnet**, daß die Ablenkeinrichtung mindestens einen bewegbaren Optikteil (20, 22; 70; 100) im Strahlengang des Laserstrahls aufweist, der den Laserstrahl nach einem vorbestimmten Austastmuster über die Strahlfleckfläche bewegt.

9. Vorrichtung nach Anspruch 8,
dadurch **gekennzeichnet**, daß die Ablenkeinrichtung eine Taumeleinheit ist.

10. Vorrichtung nach Anspruch 9,
dadurch **gekennzeichnet**, daß die Taumeleinheit um eine erste Achse eine Drehbewegung (78) und um eine zweite Achse eine Kippbewegung (72) ausführt.

11. Vorrichtung nach einem der Ansprüche 8 bis 10,
dadurch **gekennzeichnet**, daß der Optikteil zwei übereinander angeordnete schwenkbare Planplatten (20, 22) aufweist, deren Schwenkachsen einen Winkel einschließen, und daß eine Antriebseinrichtung (28, 30) vorgesehen ist, die die Planplatten phasenverschoben um ihre Schwenkachsen schwenkt.

12. Vorrichtung nach Anspruch 1,
dadurch **gekennzeichnet**, daß die beiden Planplatten (20, 22) einen Winkel von 90° einschließen, und daß die Antriebseinrichtung zwei Winkelmotoren (28, 30) aufweist, die mit um 90° gegeneinander verschobenen Sinus-Signalen angesteuert werden.

13. Vorrichtung nach einem der Ansprüche 8 bis 10,
dadurch **gekennzeichnet**, daß der Optikteil ein Spiegel (70) ist.

14. Vorrichtung nach einem der Ansprüche 8 bis 10,
dadurch **gekennzeichnet**, daß der Optikteil eine Planplatte (100) ist.

15. Vorrichtung nach einem der Ansprüche 8 bis 10, 13 oder

dadurch **gekennzeichnet**, daß der Optikteil (70, 100) um zwei Schwenkachsen verschwenkbar ist.

16. Vorrichtung nach einem der Ansprüche 8 bis 15,
dadurch **gekennzeichnet**, daß ein drehzahlgeregelter Motor (28, 30; 74) den Optikteil (20, 22; 70; 100) um die eine Schwenkachse dreht.

17. Vorrichtung nach einem der Ansprüche 8 bis 10, 15 oder 16,
dadurch **gekennzeichnet**, daß ein weiterer Antriebsmotor (124) für die Kippbewegung des Optikteils um die andere Schwenkachse vorgesehen ist.

18. Vorrichtung nach einem der Ansprüche 8 bis 10 oder 15 bis 17,
dadurch **gekennzeichnet**, daß der Optikteil (70; 100) um die andere Schwenkachse manuell kippbar ist.

19. Vorrichtung nach Anspruch 8,
dadurch **gekennzeichnet**, daß der Optikteil zwei Spiegel aufweist, deren Schwenkachsen zur zeilenförmigen Abtastung der Strahlfleckfläche einen 90°-Winkel einschließen, oder die zur kreisringförmigen Abtastung in Richtung des Laserstrahls schwingen.

20. Vorrichtung nach einem der Ansprüche 8 bis 10 oder 15 bis 19,
dadurch **gekennzeichnet**, daß für die Bewegung des Optikteils (100) eine Magnetanordnung (152, 154, 158) vorgesehen ist.

21. Vorrichtung nach Anspruch 20,
dadurch **gekennzeichnet**, daß die Halterung (146, 146'') des Optikteils (100) eine erste Magnetanordnung (152) aufweist und daß eine zweite, stationäre Einzelmagnetelemente aufweisende Magnetanordnung (158) vorgesehen ist, die mit der ersten Magnetanordnung (152) zusammen wirkt, wobei die einzelnen Magnetelemente (158) zyklisch an- und abstoßend sind und eine Taumelbewegung des Optikteils erzeugen.

22. Vorrichtung nach Anspruch 21,
dadurch **gekennzeichnet**, daß bezüglich der ersten Magnetanordnung (152) entgegengesetzt zur zweiten Magnetanordnung (158) eine dritte, stationäre Magnetanordnung (154) vorgesehen ist, die die erste Magnetanordnung (152) abstößt.

23. Vorrichtung nach Anspruch 22,
dadurch **gekennzeichnet**, daß die erste und die dritte Magnetanordnung (152,154) aus Permanentmagneten bestehen und daß die Einzelmagnetelemente (158) der zweiten Magnetanordnung Spulen sind.

24. Vorrichtung nach Anspruch 23,
dadurch **gekennzeichnet**, daß die Anzahl der Spulen (158) drei oder vier ist und daß den Spulen jeweils phasenverschoben ein Strom aufgeprägt wird.

25. Vorrichtung nach einem der Ansprüche 1 bis 24,
dadurch **gekennzeichnet**, daß ein Ziel-Lichtstrahl auf einer den zu bearbeitenden Fleck umgebenden Bahn bewegt wird.

26. Vorrichtung nach Anspruch 25,
dadurch **gekennzeichnet**, daß insbesondere bei Verwendung eines Lasers, der Licht im nichtsichtbaren Teil des Spektrums emittiert, ein zusätzlicher Ziellaser als Lichtquelle für den Ziel-Lichtstrahl verwendet wird.

27. Vorrichtung nach Anspruch 26,
dadurch **gekennzeichnet**, daß der Laser ein Helium-Neon-Laser ist.

28. Vorrichtung nach einem der Ansprüche 25 bis 27,
dadurch **gekennzeichnet**, daß der Strahl der Ziellichtquelle auf einer Kreisringbahn geführt wird, die den zu bearbeitenden, im wesentlichen kreisförmigen Fleck konzentrisch umgibt.

29. Vorrichtung nach einem der Ansprüche 1 bis 28,
dadurch **gekennzeichnet**, daß im Strahlengang ein schneller Verschluß vorgesehen ist.

**Claims**

1. Device for the performance of coagulations on the fundus with a laser beam source,
a focusing unit (60; 60', 60'') for the focusing of the laser beam at a focusing spot of predetermined size on the fundus, said unit being designed in such a way that the aperture angle of the beam path in the area before the focusing spot ensures an energy density of the beam in this area which is not damaging to the eye,
and a deflection unit (20, 22; 70; 100; 200, 201) for the generation of a scan movement of the focusing spot along a selected path in a selected treatment area on the fundus,
characterised by the fact that means are provided to control the energy deposited on a coagulated surface element in dependence on the momentary scan location of the treatment area.

2. Device according to claim 1,
characterised by the fact that the means to control the energy deposited possess means to control the scan speed in dependence on the momentary scan location of the treatment area.

3. Device according to claim 1,
characterised by the fact that the deflector unit moves the laser beam over the desired beam spot area line by line.

4. Device according to claim 1,
characterised by the fact that the deflector device moves the laser beam over the desired beam spot area in a circular ring path.

5. Device according to claims 3 or 4,
characterised by the fact that to generate a predeterminable power density profile the means to control the energy deposited possess means to change the power of the laser beam in dependence on the location.

6. Device according to claim 5,
characterised by the fact that the power of the laser beam is increased with increasing circular ring diameter.

7. Device according to claims 4 to 6,
characterised by the fact that the revolutions of the circular movement of the laser beam are decreased with increasing circular ring diameter.

8. Device according to any of claims 1 to 7,
characterised by the fact that the deflector device has at least one movable optical part (20, 22; 70; 100) in the beam path of the laser beam which moves the laser beam over the beam spot area in a predetermined scanning pattern.

9. Device according to claim 8,
characterised by the fact that the deflector device is a tumbling unit.

10. Device according to claim 9,
characterised by the fact that the tumbling unit performs a rotation movement (78) through a first axis and a tilt movement (72) through a second axis.

11. Device according to any of claims 8 to 10,
characterised by the fact that the optical part has two swivel plano plates (20, 22) positioned one above the other whose swivel axes form an angle and by the fact that a drive device (28,30) is provided which swivels the plano plates out-of-phase around their swivel axes.

12. Device according to claim 11,
characterised by the fact that the two plano plates (20, 22) form an angle of 90° and that the drive device has two angle engines (28, 30) which are controlled with sinus signal in phase quadrature to each other.

13. Device according to any any of claims 8 to 10,
characterised by the fact that the optical part is a mirror (70).

14. Device according to any of claims 8 to 10,
characterised by the fact that the optical part is a plano plate (100)

15. Device according to any of claims 8 to 10, 13 or 14,
characterised by the fact that the optical part (70, 100) can be swivelled through two swivel axes.

16. Device according to any of claims 8 to 15,
characterised by the fact that an engine with revolution control (28, 30; 74) rotates the optical part (20, 22; 70; 100) through the one swivel axis.

17. Device according to any of claims 8 to 10, 15 or 16,
characterised by the fact that another drive engine (124) is provided for the tilt movement of the optical part through the other swivel axis.

18. Device according to any of claims 8 to 10 or 15 to 17,
characterised by the fact that the optical part (70; 100) can be tilted manually through the other swivel axis.

19. Device according to claim 8,
characterised by the fact that the optical part possesses two mirrors whose swivel axes form an angle of 90° for the line-by-line scanning of the beam spot area or which oscillate in the direction of the laser beam for a circular, ring-shaped scanning.

20. Device according to any of claims 8 to 10 or 15 to 19,

characterised by the fact that a magnet arrangement (152, 154, 158) is provided for the movement of the optical part (100).

21. Device according to claim 20, characterised by the fact that the holder (146, 146'') of the optical part (100) possesses a first magnet arrangement (152) and that a second magnet arrangement (158) with stationary single magnet elements is provided which works together with the first magnet arrangement (152) and where the single magnet elements (158) are cyclically attracting and repulsing and generate a tumbling motion of the optical part.

22. Device according to claim 21, characterised by the fact that with reference to the first magnet arrangement (152) a third, stationary magnet arrangement (154) is provided in opposition to the second magnet arrangement (158) which third magnet arrangement (154) repels the first magnet arrangement (152).

23. Device according to claim 22, characterised by the fact that the first and the third magnet arrangements (152, 154) consist of permanent magnets and that the single magnet elements (158) of the second magnet arrangement are coils.

24. Device according to claim 23, characterised by the fact that the number of the coils (158) is three or four and that the coils are impressed with a current out of phase to each other.

25. Device according to any of claims 1 to 24, characterised by the fact that a target light beam is moved on a path surrounding the spot to be processed.

26. Device according to claim 25, characterised by the fact that in particular when a laser is used which emits light in the non-visible part of the spectrum, a additional target laser is used as a light source for the target light beam.

27. Device according to claim 26, characterised by the fact that the laser is a helium-neon laser.

28. Device according to any of claims 25 to 27, characterised by the fact that the beam of the target light source is guided on a circular ring path which surrounds the substantially circular spot to be processed concentrically.

29. Device according to any of claims 1 to 28, characterised by the fact that a fast shutter is provided in the beam path.

**Revendications**

1. Dispositif pour effectuer des coagulations sur le fond de l'oeil, ce dispositif comportant une source de rayon laser, une installation de focalisation (60 ; 60', 60'') pour focaliser le rayon laser en un foyer de grandeur prédéterminée sur le fond de l'oeil, l'installation étant agencée de façon que l'angle d'ouverture du trajet des rayons dans la zone précédant la tache de focalisation garantisse dans cette zone une densité d'énergie du rayonnement ne pouvant pas occasionner des dégâts à l'oeil, et une installation de déviation ou de balayage (20, 22 ; 70 ; 100 ; 200, 201) pour produire un mouvement d'exploration de la tache de focalisation le long d'un trajet choisi dans un domaine choisi de traitements sur le fond de l'oeil, dispositif caractérisé en ce que des moyens sont prévus pour commander l'énergie appliquée sur un élément de surface coagulée, en fonction de l'endroit d'exploration momentanée du domaine à traiter.

2. Dispositif selon la revendication 1, caractérisé en ce que les moyens pour commander l'énergie appliquée présentent des moyens pour commander la vitesse d'exploration en fonction de l'endroit momentané de détection du domaine à traiter.

3. Dispositif selon la revendication 1, caractérisé en ce que le dispositif de balayage déplace le rayon laser linéairement sur la surface de tache de rayon voulue.

4. Dispositif selon la revendication 1, caractérisé en ce que l'installation de déviation et de balayage déplace le rayon laser en un trajet en couronne sur la surface de tache de rayon voulue.

5. Dispositif selon la revendication 3 ou 4, caractérisé en ce que, pour produire un profil prévisible de densité de charge, les moyens destinés à commander l'énergie appliquée présentent des moyens pour modifier, en fonction de l'endroit (à traiter) la charge de puissance du rayon laser.

6. Dispositif selon la revendication 5, caractérisé en ce que la charge de puissance du rayon laser est augmentée quand le diamètre de la couronne augmente.

7. Dispositif selon la revendication 4 à 6, caractérisé en ce que la vitesse de rotation du mouvement circulaire de rayon laser diminue avec l'augmentation du diamètre de la couronne (balayée).

8. Dispositif selon l'une des revendications 1 à 7, caractérisé en ce que l'installation de balayage présente au moins une partie d'optique (20, 22 ; 70 ; 100) mobile dans le trajet du rayon laser, partie qui déplace le rayon laser selon un modèle d'exploration déterminé sur la surface de la tache formée par le rayon.

9. Dispositif selon la revendication 8, caractérisé en ce que l'installation de balayage est une unité de nutation.

10. Dispositif selon la revendication 9, caractérisé en ce que l'unité de nutation effectue un mouvement de rotation (78) autour d'un premier axe et un mouvement de bascule (72) autour d'un second axe.

11. Dispositif selon l'une des revendications 8 à 10, caractérisé en ce que la partie optique présente deux plaques planes (20, 22) disposées l'une au-dessus de l'autre, pouvant pivoter et dont les axes de pivotement englobent un angle, et en ce qu'une installation (28, 30) d'entraînement est prévue qui provoque, avec un décalage de phases, le pivotement des plaques planes autour de leurs axes de pivotement.

12. Dispositif selon la revendication 11, caractérisé en ce que les deux plaques planes (20, 22) englobent un angle de 90° et en ce que le dispositif d'entraînement comporte deux moteurs (28, 30) agencés selon un certain angle et qui sont commandés par des signaux en forme de sinus décalés l'un de l'autre de 90°.

13. Dispositif selon l'une des revendications 8 à 10, caractérisé en ce que la pièce d'optique est un miroir (70).

14. Dispositif selon l'une des revendications 8 à 10, caractérisé en ce que la pièce d'optique est une plaque plane (100 ).

15. Dispositif selon l'une des revendications 8 à 10, 13 ou 14, caractérisé en ce que la pièce d'optique (70, 100) peut pivoter autour de deux axes de pivotement.

16. Dispositif selon l'une des revendications 8 à 15, caractérisé en ce qu'un moteur (28, 30 ; 74) à vitesse de rotation réglée fait tourner la pièce d'optique (20, 22 ; 70 ; 100) autour de l'un des axes de pivotement.

17. Dispositif selon l'une des revendications 8 à 10, 15 ou 16, caractérisé en ce qu'un autre moteur (124) est prévu pour l'entraînement du mouvement de bascule de la pièce d'optique autour de l'autre axe de pivotement.

18. Dispositif selon l'une des revendications 8 à 10 ou 15 à 17, caractérisé en ce que la pièce d'optique (70 ; 100) peut pivoter manuellement autour de l'autre axe de pivotement.

19. Dispositif selon la revendication 8, caractérisé en ce que la pièce d'optique présente deux miroirs dont les axes de basculement englobent un angle de 90° pour l'exploration linéaire de la surface de tache de rayonnement, ou qui pivotent pour une exploration en couronne dans la direction du rayon laser.

20. Dispositif selon l'une des revendications 8 à 10 ou 15 à 19, caractérisé en ce qu'un agencement à aimant (152, 154, 158) est prévu pour le déplacement de la pièce (100) d'optique.

21. Dispositif selon la revendication 20, caractérisé en ce que le support (146, 146'') de la pièce (100) d'optique présente un premier agencement à aimant (152) et en ce qu'est prévu un second agencement à aimant (158) présentant des éléments magnétiques individuels fixes et qui coopère avec le premier dispositif (152) à aimant, les divers éléments magnétiques (158) s'attirant et se repoussant de façon cyclique et provoquant un mouvement de nutation de la pièce d'optique.

22. Dispositif selon la revendication 21, caractérisé en ce que, disposé à l'opposé du second dispositif à aimant (158) par rapport au premier dispositif à aimant (152), un troisième dispositif fixe (154) à aimant est prévu et repousse le premier dispositif (152) à aimant.

23. Dispositif selon la revendication 22, caractérisé en ce que le premier et le troisième agencement 152, 154) à aimant consistent en des aimants permanents et en ce que les divers éléments magnétiques (158) du second agencement d'aimant sont des bobines.

24. Dispositif selon la revendication 23, caractérisé en ce que le nombre des bobines (158) est de trois ou quatre et en ce qu'un courant est appliqué, avec chaque fois un décalage de phases, aux bobines.

25. Dispositif selon l'une des revendications 1 à 24, caractérisé en ce qu'un rayon de lumière cible est déplacé sur un trajet entourant la tache à traiter.

26. Dispositif selon la revendication 25, caractérisé en ce qu'en particulier lorsqu'on utilise un laser émettant de la lumière dans la partie non visible du spectre, on utilise un laser cible supplémentaire comme source de lumière pour le rayon de lumière cible.

27. Dispositif selon la revendication 26, caractérisé en ce que le laser est un laser à hélium-néon.

28. Dispositif selon l'une des revendications 25 à 27, caractérisé en ce que le rayon de la source de lumière cible est guidé sur un trajet en couronne qui entoure concentriquement la tache essentiellement circulaire à traiter.

29. Dispositif selon l'une des revendications 1 à 28, caractérisé en ce que, sur le trajet du rayonnement, est prévu un obturateur rapide.

**10** **34** **10** **20** **20** **24** **28** **22** **22** **36** **26** **30** **12** **32**

x (sin)

± 10°

± 10°

y (cos)

x   y

(a)       (b)

Fig. 1

Fig 2a

Fig 2b

**Fig. 3**

Fig. 4

Fig. 5

Fig. 6a

Fig. 6b

Fig. 6c

Fig. 7

Fig. 8